# EUROPEAN PATENT SPECIFICATION

(11) **EP 2 149 602 B1**
(45) Date of publication and mention of the grant of the patent: **17.12.2014**
(21) Application number: 08740507.2
(22) Date of filing: 16.04.2008
(51) Int. Cl.: C12N 5/073, C12N 5/0775

(54) **METHOD FOR CONCENTRATION OF SIDE POPULATION CELL FRACTION**
VERFAHREN ZUR KONZENTRATION EINER NEBENPOPULATION-ZELLFRAKTION
PROCÉDÉ DE CONCENTRATION DE FRACTIONS DE CELLULES DE POPULATION LATÉRALE

(30) Priority: 17.04.2007 JP 2007107812
(43) Date of publication of application: 03.02.2010
(73) Proprietor: Nidek Co., Ltd., Aichi 443-0038 (JP)
(72) Inventor: YOKOYAMA, Yasunobu, Hachioji-shi Tokyo 192-0916 (JP); SAKURAGAWA, Norio, Kodaira-shi Tokyo 187-0032 (JP)
(74) Representative: Kador & Partner
(86) International application number: PCT/JP2008/057428
(87) International publication number: WO 2008/133140

(56) References cited:
- JP-A- 2004 254 682
- US-A1- 2002 045 260
- LIN K K ET AL: "Purification of hematopoietic stem cells using the side population." METHODS IN ENZYMOLOGY 2006 LNKD- PUBMED:17161700, vol. 420, 2006, pages 255-264, XP9134449 ISSN: 0076-6879
- CHALLEN GRANT A ET AL: "A side order of stem cells: The SP phenotype" STEM CELLS, ALPHAMED PRESS, DAYTON, OH, US LNKD- DOI:10.1634/STEMCELLS.2005-0116, vol. 24, no. 1, 1 January 2006 (2006-01-01), pages 3-12, XP002562631 ISSN: 1066-5099
- KESSINGER A. ET AL.: 'An ex vivo model of hematopoietic stem cell mobilization' CYTOTHERAPY vol. 7, no. 6, 2005, pages 463 - 469, XP009134451
- HUNG S.-C. ET AL.: 'Isolation and characterization of size-sieved stem cells from human bone marrow' STEM CELLS vol. 20, 2002, pages 249 - 258, XP009054150
- UMEMOTO T. ET AL.: 'Limbal epithelial side-population cells have stem cell-like properties, including quiescent state' STEM CELLS vol. 24, 2006, pages 86 - 94, XP009134453
- REDVERS R.P. ET AL.: 'Side population in adult murine epidermis exhibits phenotypic and functional characteristics of keratinocyte stem cells' PROC. NATL. ACAD. SCI. USA vol. 103, no. 35, 2006, pages 13168 - 13173, XP008125299
- PARK K.-S. ET AL.: 'The side population cells in the rabbit limbus sensitively increased in response to the central cornea wounding' INVEST. OPHTHALMOL. VIS. SCI. vol. 47, no. 3, 2006, pages 892 - 900, XP008125246

## Description

### Technical Field

The present invention relates to a method of enriching a side population cell fraction. The method according to the present invention makes it possible to highly enrich a multipotent side population cell fraction in a simple manner and can be readily used in the regenerative medicine.

### Background Art

In the field of the organ reconstruction, methods for cell transplantation using side population cells (hereinafter also referred to as "SP cells") with a high proliferation potency and multipotency have been studied. It has been reported that functions of the injured tissue can be recovered by transplanting a small number of SP cells therein (see, Non-patent Literatures 1 and 2). SP cells, which could excrete Hoechst 33342 fluorescent dye out of the cells, were found for the first time in murine bone marrow cells (see, Non-patent Literature 2). It is thought that SP cells excrete Hoechst 33342 out of the cells by a molecular pump represented by the protein (MDR molecule) encoded by an ABC transporter MDR (multi drug resistance gene). Further studies revealed that SP cells exist in human, simian, swine, canine, chick, quail, zebrafish and the like besides mouse, and included in tissues such as umbilical cord, skeletal muscle, mammary gland, lung, liver, epithelium, forebrain, testis, heart, kidney, limbal epithelium and prostate gland besides bone marrow (see, Non-patent Literatures 3, 4, 5, 6).

The present inventors found that SP cells derived from human amnion can be obtained in an amount large enough to provide stably and have no problem in the compatibility of the histocompatibility antigen type (hereinafter also referred to as "HLA type") when used for cell transplantation, and thus completed an invention of multipotent SP cells derived from human amnion which is useful for the regenerative medicine (see, Patent Literature 1).

However, percentage of SP cells existing in a body tissue is extremely low; they exist in bone marrow cells at a percentage of no more than about 0.1%, and in human amnion mesenchymal cell layer at a percentage of no more than about 0.2-0.6%. As mentioned above, cell transplant therapy using SP cells is regarded as a promising therapy in studies of the organ reconstruction, and it becomes an important subject to develop a method by which SP cells existing at a low percentage can be enriched.

Several methods have been reported for increasing or enriching SP cells, for example, (1) a method in which a virus vector carrying a gene such as MDR1 and Akt is introduced into murine bone marrow cells to increase SP cells (see, Non-patent Literatures 4 and 5); (2) a method in which cells are treated with anticancer agents such as mitoxantrone to enrich SP cells having a capacity to excrete such agents (see, Non-patent Literatures 6 and 7); (3) a method in which cells are gated using cell surface markers or complexity in cells to enrich SP cells by fluorescence activated cell sorter (FACS) or the like (see, Non-patent Literatures 7 and 8); (4) a method in which bone marrow cells are cultured under low oxygen condition to increase SP cell fraction (see, Non-patent Literature 9); and (5) a method in which impurities which get included when collecting cells from a tissue arc removed by density gradient centrifugation to increase a percentage of SP cell fraction (see, Non-patent Literature 10).

However, in view of the use of the enriched SP cells for cell transplant therapy, some sort of risk may occur when cells which have transgenes or arc treated with anticancer agents according to the above described methods (1) and (2) are transplanted into bodies. The above-mentioned methods (3) to (5) can be carried out in combination with the present invention to further increase the efficiency of SP cell enrichment. Therefore, it is demanded to develop a novel method for SP cell enrichment.

Patent Literature 1: JP 2004-254682 A
Non-patent Literature 1: Goodell, M.A. et al., Nat Med., 1997, Dec;3(12):1337-1345
Non-patent Literature 2: Gussoni, E. et al., Nature., 1999, Sep 23;401(6751):390-394
Non-patent Literature 3: Asakura, A. et al., Exp Hematol. 2002, Nov;30(11):1339-1345
Non-patent Literature 4: Schienda, J. et al., PNAS., 2006., Jan 24; 103(4); 945-950
Non-patent Literature 5: Challen, G.A. et al., Stem Cells., 2006, 24:3-12
Non-patent Literature 6: Patrawala, L. et al., Cancer Res., 2005, July 15;65(14): 6207-6219
Non-patent Literature 7: Bunting, K.D. et al., Blood., 2000, Aug 1;96(3):902-909
Non-patent Literature 8: Mogi, M. et al., J Biol Chem., 2003, Oct 3;278(40):39068-39075
Non-patent Literature 9: Hirschmann-Jax, C. et al., PNAS., 2004, Sep 28;101(39):14228-14233
Non-patent Literature 10: Zou, S. et al., PNAS., 2002, Sep 17;99(19):12339-12344
Non-patent Literature 11: Kotton, D.N. et al. Blood., 2005, Sep 1;106(5):1574-1580
Non-patent Literature 12: Krishnamurthy, P. et al., J Biol Chem., 2004, Jun 4;279(23):24218-24225
Non-patent Literature 13: Budak, M.T. et al., J Cell Sci, 2005, Apr 15;118(Pt 8):1715-1724

### Disclosure of the Invention

### Problems Which the Invention Tries to Solve

An object of the present invention is to provide a method by which SP cells existing in tissues at a low percentage can be enriched with a high purity in a simple manner.

### Means for Solving the Problem

The present inventors intensively studied to find that SP cells do not show migration but acquire migratory activity when they are differentiated into mature mesenchymal cells, and that SP cell fraction can be enriched based on the difference in the migratory activity between SP cells and mature mesenchymal cells, thereby completing the present invention.

That is, the present invention provides a method of enriching side population cells originated from a mesenchymal layer from an amnion, which method comprises culturing mesenchymal cells obtained from a mesenchymal layer from an amnion on a porous substrate, wherein said mesenchymal cells comprise side population cells without a migratory capacity and matured mesenchymal cells with a migratory capacity, wherein the porous substrate partitions the culture system into an upper layer fraction and a lower layer fraction, and wherein the porous substrate has penetrating pores with a size which allows the matured mesenchymal cells with a migratory capacity to pass through said pores into the lower layer fraction when the nutrient components in the upper layer fraction are continuously consumed by the metabolism of said mesenchymal cells and recovering the side population cells without a migratory capacity remaining on said porous substrate in the upper layer fraction after the culture.

### Effects of the Invention

By the present invention, a method by which SP cells can be safely enriched with a high purity in a simple manner. The method of enriching SP cells according to the present invention does not cause any biological alterations in cells, and can be carried out in combination with other methods to provide the enriched SP cells with a still higher density.

### Brief Description of the Drawings

Fig. 1 shows FACS data demonstrating the results of the enrichment of SP cells from human amniotic mesenchymal cells in Example 1. The SP cell fraction is encircled with a solid line.
Fig. 2 shows the comparison of the migratory activity between Fresh cells and Cryopreserved cells in Example 2.
Fig. 3 schematically shows the method of enriching SP cells derived from an amnion according to the present invention.

### Mode for Carrying out the Invention

The enrichment method of the present invention may be carried out by culturing SP cells without a migratory capacity together with the matured mesenchymal cells, i.e., the differentiated SP cells with a migratory capacity, on a porous substrate. That is, as concretely described in Examples below, when a mesenchymal cell layer is cultured on a porous substrate, the matured mesenchymal cells having a migratory capacity migrate through pores of a porous membrane into the lower nutrient-rich fraction of the medium below the porous membrane, whereas, SP cells not having a migratory capacity cannot migrate and thus remain in the upper medium fraction. Thus, based on such a nature of SP cells described above, SP cells may be separated from main population cells (hereinafter also referred to as "MP cells"), which occupy most of the mesenchymal cell layer, in a simple manner with a higher density.

It is expected that SP cells will be used as donor cells in the cell transplant therapy in the future. In such a case, it is thought that SP cells derived from an amnion, which are free from the problem in the compatibility of the histocompatibility antigen type, are especially suitable, and therefore an amnion is the tissue from which the mesenchymal layer is collected.

The mesenchymal layer mentioned above is not restricted as long as it is originated from those animal species belonging to Amniota, more preferably mammals. Considering the use of the present invention for the regenerative medical treatment in human, human is especially preferred in view of histocompatibility. Thus, it is most preferred to enrich SP cells derived from human amnion in cases where the present invention is used in the organ reconstruction and the regenerative medicine. Human amnions are excreted from uterus during delivery, and therefore they may be provided in a sufficient amount through medical institutions and the like.

The mesenchymal layer used in the present invention may be collected by a common method. For example, the amniotic mesenchymal layer used in the present invention may be collected by a common method in which, for example, a mesenchymal cell layer is peeled off from a chorionic layer and treated with an enzyme such as collagenase or papain (see, Patent Literature 1). The existence of the required SP cells in the collected mesenchymal layer may be confirmed by staining the layer with a fluorescent dye such as Hoechst 33342. SP cells are characterized in that the fluorescence intensity observed when staining SP cells is weaker than that observed when staining MP cells and the like, because SP cells have a high capacity to excrete Hoechst 33342 out of the cells. Fluorescence intensity of the cells stained by Hoechst 33342 may be measured by a two-dimensional analysis at wavelength of 675 nm and 450 nm by flow cytometry with FACS or the like. That is, SP cells exist in a region where fluorescence intensities at 675 nm and 450 nm are both weak. Analysis by Hoechst 33342 staining may be easily carried out according to a common method (see, Patent Literature 1 or Non-patent Literature 1).

The porous substrate used in the present invention is not restricted as long as the substrate has penetrating pores with a size which allows the cells contained in the mesenchymal layer to pass through, and the mean diameter of the pores is preferably in a range of 3 µm to 8 µm. Commercially available porous substrates may also be used in the present invention. In the Examples below, Transwell (registered trademark) having a pore diameter of 3.0 µm or 8.0 µm manufactured by Corning was used, but the present invention is not restricted thereto.

In the present invention, cells contained in the mesenchymal layer may be cultured in accordance with a conventional method for culturing the cells (sec, Patent Literature 1) except that the culture condition is different from the conventional method in that cells are adhered onto the porous substrate. Typical examples of the method include, but not limited to, a culture method in which cells are kept to adhere onto a porous substrate as well as kept to soak in a culture medium, using as a culture medium a basal medium such as Dulbecco's modified Eagle's minimal essential medium (hereinafter also referred to as "DMEM") or a liquid prepared by adding known additives to a basal medium. The time period for enrichment is also not restricted and it is preferred to continuously culture the cells for not less than 7 days, especially preferably not less than 10 days, in order to achieve a sufficient enrichment efficiency. The upper limit of the time period for the continuous culture is not restricted, and a sufficient enrichment efficiency may be achieved by culturing cells for up to 30 days. Depending on the type of SP cells, a sufficient effect may be obtained by culturing cells for up to 20 days.

In the present invention, the method for recovering cells remaining on the porous substrate after the culture is not restricted as long as the cells adhering to the porous substrate can be recovered while keeping them alive. Examples of such a method include a method in which the cells were peeled off from the porous substrate by enzyme treatment or the like, and the peeled cells are recovered by centrifugation. "After the culture" used herein means "after enriching SP cells by using the porous substrate".

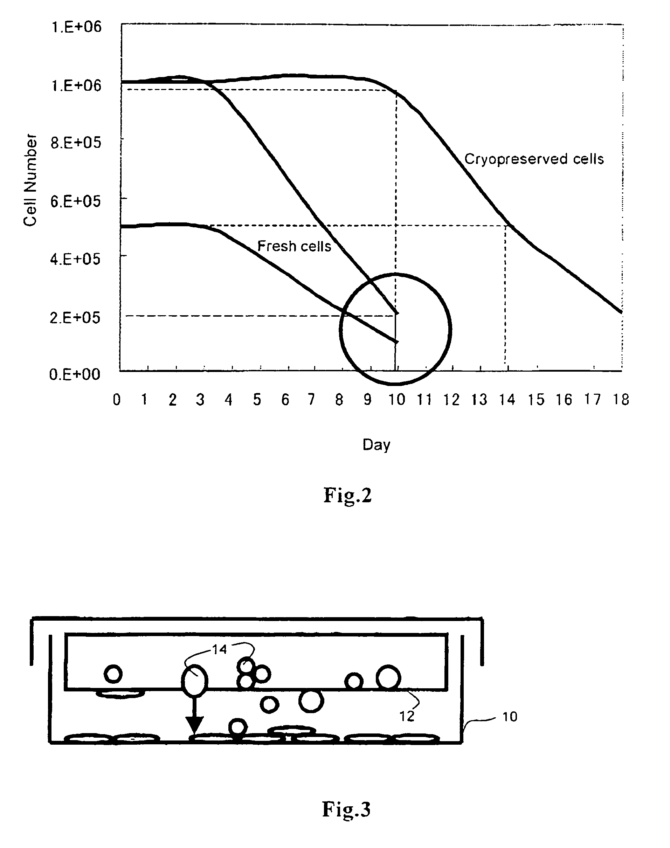

The present invention is a method of enriching SP cells based on the difference in the migratory capacity between SP cells and the matured mesenchymal cells. "Migratory capacity" used herein refers to "motility to gather or escape depending on the concentration gradient of a chemotactic factor or the like". In the present invention, the migratory capacity is thought to be as follows. The culture system in the present invention is partitioned by the porous substrate into the upper layer fraction and the lower layer fraction. In the upper layer fraction, cells are seeded at the start of the culture, and the nutrient components are continuously consumed by the metabolism of the cells, whereas, the lower layer fraction remains rich in the nutrient components. Therefore, the matured mesenchymal cells having a migratory capacity respond to the concentration gradient of the nutrient components and migrate into the lower layer fraction (see, Fig. 3). Fig. 3 schematically shows the enrichment method according to the present invention. In Fig. 3, reference numeral 10 denotes a Petri dish; reference numeral 12 denotes a porous substrate; and reference numeral 14 denotes mesenchymal cells. When mesenchymal cells are cultured on a porous substrate 12, matured mesenchymal cells having a migratory capacity pass through the pores of the porous substrate 12 to migrate to the Petri dish, whereas, SP cells remain on the porous substrate 12, and thus SP cells are enriched on the porous substrate 12.

The existence ratio of SP cells after the enrichment according to the present invention may be measured by the two-dimensional analysis using Hoechst 33342 staining as mentioned above (see, Patent Literature 1, or Non-patent Literature 1).

The present invention will now be described more concretely by way of an example thereof. However, the present invention is not restricted to the example below.

### Examples

### Example 1: Enrichment of SP Cells from Human Amniotic Mesenchymal Layer

### 1. Enrichment Culture of SP Cells

Immediately after collecting mesenchymal cells from human amnion in accordance with a common method (see, Patent Literature 1), the cells were seeded onto a polycarbonate membrane Transwell (registered trademark) with a pore diameter of 3.0 µm manufactured by Corning at a cell density of 1.13x10⁵ (cells/cm²), and cultured in DMEM/F-12 (1:1) supplemented with 20% fetal bovine serum (FBS) at 37°C for 10 days under 5% CO₂. As the culture medium turned yellow from 7 days after the start of the culture, the medium was replaced with a new one every 24 hours from 7 days after the start of the culture.

### 2. Separation of SP Cells

(1) The above-mentioned Transwell (registered trademark) was taken out and placed in a new Petri dish.
(2) The culture medium above the membrane of Transwell (registered trademark) was recovered.
(3) PBS (-) was added to the upper and lower surfaces of the membrane mentioned above, and cells adhering on the membrane were recovered by pipetting. PBS (-) remaining on the lower surface was removed by an aspirator. This operation was repeated 3 times.
(4) A mixed solution of 0.02% trypsin solution/0.01% EDTA was added to the upper and lower surfaces of the membrane, and the membrane was treated at 37°C for 2 minutes under 5% CO₂.
(5) Cells adhering on the upper surface of the membrane were further detached by pipetting and recovered in the preliminarily prepared DMEM/F-12 (1:1) culture medium supplemented with 20% FBS.
(6) The above described operations (4) and (5) were repeated 3 times.
(7) For Hoechst 33342 staining, an aliquot (in small amount) of the cells recovered by the operations described above was taken to count the viable cells.
(8) The recovered cells were centrifuged at 1000 rpm at room temperature for 5 minutes to form a pellet.
(9) The supernatant was removed off by an aspirator, and the viable cells were suspended in DMEM/F-12 (1:1) culture medium supplemented with 5% FBS. The cell density was adjusted to 5 x 10⁵ cells/ml.
(10) Cells in a population of 1 x 10⁶ cells were transferred to a Petri dish and cultured under shaking at 37°C for 60 minutes under 5% CO₂ in an incubator.
(11) Hoechst 33342 was added to the culture medium of the cells cultured under shaking as described above at a final concentration of 1.5 µg/ml (667-fold dilution).
(12) The cells were further cultured under shaking at 37°C for 90 minutes under 5% CO₂ in an incubator so that Hoechst 33342 was incorporated into the cells.
(13) An equal volume of cold HBSS+ (Hanks Balanced Salt Solution manufactured by Gibco supplemented with 2%FCS, 10mM HEPES buffer manufactured by Gibco) was added to the culture medium described above.
(14) The cells described above were transferred to a tube containing 10 ml of cold HBSS+ through a cell strainer.
(15) Furthermore, the Petri dish used in the above-described shaking culture was washed with cold HBSS+ again, and the cold HBSS+ was recovered and made to pass through a cell strainer to completely recover the cells.
(16) The recovered cells were centrifuged at 1000 rpm at room temperature for 5 minutes to form a pellet.
(17) While keeping the cells at 4°C, the cell density was adjusted to 1 x 10⁴ cells/ml by adding thereto cold HBSS+ containing of PI (propidium iodide) at the final concentration of 2 µg/mL.
(18) An aliquot of the cell suspension was taken to count the viable cells.
(19) Using EPICS ALTRA HyPcrSort (Beckman Coulter), Hoechst 33342 dye was excited by UV laser at 350 nm, and Hoechst Blue/350nm and Hoechst Red/675nm were detected, thereby alanyzing and recovering the SP fraction.

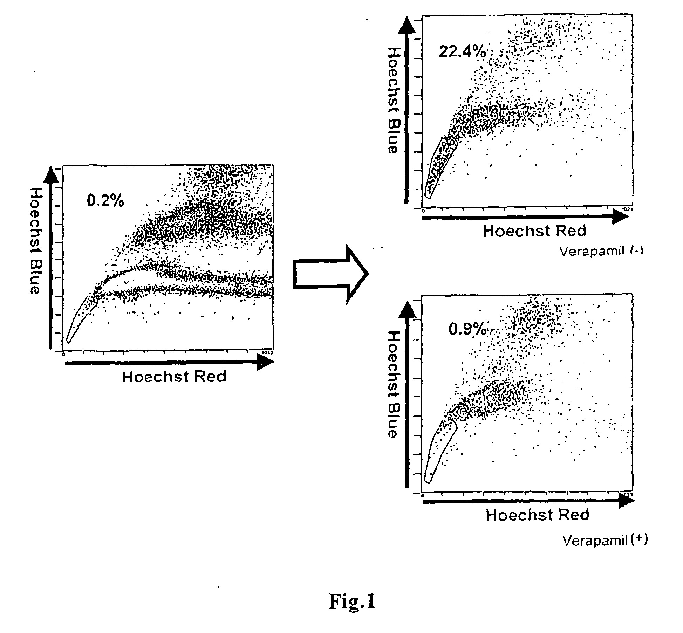

The results of FACS is shown in Fig. 1. A part encircled with a solid line is the SP cell region. When mesenchymal cells were stained with IIoechst 33342 right after collecting them from an amnion and subjected to two-dimensional fluorescence analysis by FACS in the same manner as described above, the existence ratio of SP cells was 0.2% in the total cells. In contrast, when SP cells were enriched by using Transwell (registered trademark), the existence ratio of SP cells was 22.4% in the total cells, confirming that the density of SP cells was enriched to more than 100-fold (see, Fig. 1). When verapamil, which is an inhibitor of the functions of MDR molecule, was added to the SP cell fraction (indicated as "Verapamil (+)" in Fig. 1), the existence ratio of the cells was decreased to 0.9%. Thus, it was confirmed that a cell population contained in this SP cell fraction was SP cells.

### Example 2: Comparison of Efficiency of SP Cell Enrichment between Mesenchymal Cells Right After Collection from Human Amnion and Those Cryopreserved and Thawed

In the same manner as in Example 1 above, mesenchymal cells right after collection from human amnion (Fresh cells) and those cryopreserved after collection from human amnion and then thawed (Cryopreserved cells) were cultured on Transwell (registered trademark), and cells adhering on the upper surface of the membrane of Transwell (registered trademark) were counted with time after the start of the culture. Fresh cells and Cryopreserved cells were cultured and recovered in the same manner as in Example 1, except that the time period for culturing cells on Transwell (registered trademark) was extended to 14 days in consideration of the time period until the frozen-thawed cells recovered normal metabolic functions.

Fig. 2 shows the number of Fresh cells and Cryopreserved cells adhering to the upper surface of the membrane of Transwell (registered trademark) after culturing cells on Transwell (registered trademark) for a prescribed time. As shown in Fig. 2, the number of Fresh cells adhering to the upper surface of the membrane began to decrease around Day 3 from the start of the culture, and decrease to about 1/5 on Day 10. On the other hand, in the case of Cryopreserved cells, there was almost no change in the number of the cells on Day 7 from the start of the culture, and the cell number decreased to about 1/2 on Day 14, indicating that the number of Cryopreserved cells began to decrease much later than that of fresh cells. The reason of this delay is thought to be as follows. Fresh cells already had a high metabolic functions at the start of the culture, and thus the matured mesenchymal cells contained in Fresh cells could migrate to the layer below the membrane by their migratory capacity at a relatively early stage, whereas, the metabolic functions of Cryopreserved cells were low at the start of the culture, and thus it took considerably more time for the matured mesenchymal cells contained in Cryopreserved cells to acquire the metabolic functions required to migrate to the layer below the membrane by the migratory capacity. If the matured mesenchymal cells had moved to the lower layer of Transwell (registered trademark) through penetrating pores by the gravity, such a time difference would not have observed between Fresh cells and Cryopreserved cells. Thus, this experiment revealed that matured mesenchymal cells migrate to the layer below the membrane by their own migratory capacity.

### Example 3: Comparison of Transwell (Registered Trademark) with Different Pore Sizes

In the same manner as in Example 2 above, Fresh cells were cultured on Transwell (registered trademark) with a pore diameter of 3.0 µm or 8.0 µm, and cells adhering to the upper surface of the membrane were counted to investigate the influence of the pore diameter on cell migratory activity.

The comparing investigation using Transwell (registered trademark) with different pore sizes revealed that the migration rate of the cells was not affected by pore diameter between 3.0 µm and 8.0 µm.

### Example 3: Comparison of Numbers of Cells Seeded on Transwell (Registered Trademark)

In the same manner as in Example 2, Fresh cells were seeded on Transwell I (registered trademark) at a cell density of I x 10⁶, 5 x 10⁶ or 1x10⁷ cells, and the cells adhering to the upper surface of the membrane were counted to investigate the influence of the initial cell number on the cell migratory activity.

The comparing investigation using various initial cell number revealed that the migration rate was not affected by the initial cell number between 1 x 10⁶ to 1 x 10⁷. The number of the cells recovered from SP cell fraction adhering to the upper surface of the membrane depended on the initial cell number.

## Claims

1. A method of enriching side population cells originated from a mesenchymal layer from an amnion, which method comprises
culturing mesenchymal cells obtained from a mesenchymal layer from an amnion on a porous substrate,
wherein said mesenchymal cells comprise side population cells without a migratory capacity and matured mesenchymal cells with a migratory capacity,
wherein the porous substrate partitions the culture system into an upper layer fraction and a lower layer fraction, and
wherein the porous substrate has penetrating pores with a size which allows the matured mesenchymal cells with a migratory capacity to pass through said pores into the lower layer fraction when the nutrient components in the upper layer fraction are continuously consumed by the metabolism of said mesenchymal cells,
and recovering the side population cells without a migratory capacity remaining on said porous substrate in the upper layer fraction after the culture.

2. The method according to claim 1, wherein the penetrating pores of said porous substrate have an average diameter of 3 µm to 8 µm.

3. The method according to claim 1 or 2, wherein said culture is carried out for not less than 10 days.

4. The method according to any one of claims 1 to 3, wherein said mesenchymal layer is from a mammal.

5. The method according to any one of claims 1 to 4, wherein said mesenchymal layer is from human.

## Patentansprüche

1. Verfahren zur Anreicherung von Randpopulationszellen [Engl.: side population cells; SP-cells], die aus einer Mesenchymschicht von einem Amnion stammen, wobei das Verfahren umfasst
Kultivieren der Mesenchymzellen, die aus einer Mesenchymschicht von einem Amnion erhalten werden, auf einem porösen Substrat,
wobei die besagten Mesenchymzellen Randpopulationszellen, die keine Migrationsfähigkeit haben, und ausgereifte Mesenchymzellen, die eine Migrationsfähigkeit haben, umfassen,
wobei das poröse Substrat das Kultursystem in einen oberen Schichtteil und einen unteren Schichtteil unterteilt, und
wobei das poröse Substrat Penetrationsporen mit einer Größe hat, die es den ausgereiften Mesenchymzellen, die eine Migrationsfähigkeit haben, erlaubt durch die besagten Poren in den unteren Schichtteil überzugehen, wenn die Nährstoffbestandteile in dem oberen Schichtteil kontinuierlich durch den Stoffwechsel der besagten Mesenchymzellen verbraucht werden,
und Gewinnen der Randpopulationszellen, die keine Migrationsfähigkeit haben und die nach der Kultur auf dem besagten porösen Substrat in dem oberen Schichtteil zurückbleiben.

2. Verfahren nach Anspruch 1, wobei die Penetrationsporen des besagten porösen Substrats einen durchschnittlichen Durchmesser von 3 µm bis 8 µm haben.

3. Verfahren nach Anspruch 1 oder 2, wobei die besagte Kultur für nicht weniger als 10 Tage durchgeführt wird.

4. Verfahren nach einem der Ansprüche 1 bis 3, wobei die besagte Mesenchymschicht von einem Säugetier ist.

5. Verfahren nach einem der Ansprüche 1 bis 4, wobei die besagte Mesenchymschicht von einem Menschen ist.

## Revendications

1. Procédé destiné à enrichir des cellules d'une population latérale issues d'une couche mésenchymateuse en provenance d'un amnios, lequel procédé comprend les étapes consistant à :
mettre en culture des cellules mésenchymateuses obtenues à partir d'une couche mésenchymateuse en provenance d'un amnios sur un substrat poreux ;
dans lequel lesdites cellules mésenchymateuses comprennent des cellules de population latérale sans capacité de migration et des cellules mésenchymateuses mûries qui présentent une capacité de migration ;
dans lequel le substrat poreux partitionne le système de culture en une fraction de couche supérieure et en une fraction de couche inférieure ; et
dans lequel le substrat poreux présente des pores de pénétration dont la taille permet aux cellules mésenchymateuses mûries qui présentent une capacité de migration, de passer à travers lesdits pores dans la fraction de couche inférieure lorsque les composants nutritifs dans la fraction de couche supérieure sont consommés de manière continue par le métabolisme desdites cellules mésenchymateuses ;
et récupérer lesdites cellules de population latérale sans capacité de migration qui restent sur ledit substrat poreux dans la fraction de couche supérieure après la culture.

2. Procédé selon la revendication 1, dans lequel les pores de pénétration dudit substrat poreux présentent un diamètre moyen compris entre 3 µm et 8 µm.

3. Procédé selon la revendication 1 ou la revendication 2, dans lequel ladite culture est exécutée au cours d'une période qui n'est pas inférieure à 10 jours.

4. Procédé selon l'une quelconque des revendications 1 à 3, dans lequel ladite couche mésenchymateuse provient d'un mammifère.

5. Procédé selon l'une quelconque des revendications 1 à 4, dans lequel ladite couche mésenchymateuse provient d'un être humain.
